# EUROPEAN PATENT APPLICATION

(11) **EP 4 052 731 A1**
(43) Date of publication of application: **07.09.2022**
(21) Application number: 20881085.3
(22) Date of filing: 14.10.2020
(51) Int. Cl.: A61L 2/10, C02F 1/32

(54) **FLUID STERILIZATION DEVICE**

(30) Priority: 30.10.2019 JP 2019197353
(71) Applicant: STANLEY ELECTRIC CO., LTD., Tokyo 153-8636 (JP)
(72) Inventor: TANAKA Hideaki, Tokyo 153-8636 (JP); KATO Hiroyuki, Tokyo 153-8636 (JP); SHINNO Kazuhisa, Tokyo 153-8636 (JP)
(74) Representative: Schmidbauer, Andreas Konrad
(86) International application number: PCT/JP2020/038823
(87) International publication number: WO 2021/085143

(57) **Abstract**

A fluid sterilization device 1 includes: a cylindrical body 5c of a housing 5 having a flow passage through which fluid flows in axial direction; inflow port 5a through which the fluid flows into the cylindrical body 5c; outflow port 5d on the outer circumference of the cylindrical body 5c; light source 3 provided on an end section on opposite side to the inflow port 5a; and quartz cap 9 composed of a cylindrical section 9a and a leading end section 9b, and transmits, collects, or scatters ultraviolet light. In the quartz cap 9, the cylindrical section 9a is fitted to an inner wall of the cylindrical body 5c, and a boundary section 9c is disposed where coinciding with an end surface X of the outflow port 5d on the side close to the light source 3 or where the boundary section 9c protrudes beyond the end surface X.

## Description

### Technical Field

The present invention relates to a fluid sterilization device that sterilizes, by ultraviolet light, a fluid flowing through a flow passage.

### Background Art

In recent years, the bactericidal action of ultraviolet rays has been used in food storage germicidal lamps and medical devices. Equipment is also well known that uses ultraviolet LEDs to irradiate ultraviolet light on a fluid flowing through a flow passage to sterilize the fluid so as to use the sterilized fluid as cleaning water or the like.

For example, the fluid sterilization module of Patent Literature 1 below includes a flow passage pipe having an internal space through which running water flows, and a light source that protrudes into the internal space from the side of one end of the flow passage pipe and can irradiate ultraviolet rays toward the internal space.

Specifically, the light source includes a heat dissipating member that dissipates heat generated, and one end section of the heat dissipating member is placed in such a manner as to protrude beyond a first inflow/outflow port toward the other end section of the flow passage pipe. Further, the heat dissipating member has a columnar section that serves as both a light source mounting section on which the light source is mounted and a heat dissipating section that dissipates the heat generated by the light source (Patent Literature 1 / Paragraphs 0012 and 0016, and FIG. 1).

### Citation List

### Patent Literature

Patent Literature 1: Japanese Patent Application Laid-Open No. 2019-18198

### Summary of Invention

### Technical Problem

However, in the fluid sterilization module of Patent Literature 1, depending on the placement direction or the direction of running water, air bubbles are generated in the internal space between the columnar section and the inner wall of the module, so that the sterilization efficiency by ultraviolet rays may decrease.

The present invention has been made in view of the circumstances described above, and an object of the invention is to provide a fluid sterilization device capable of increasing the utilization efficiency of ultraviolet light and improving sterilization effect.

### Solution to Problem

A fluid sterilization device in accordance with the present invention includes: a cylindrical body that has a flow passage through which a fluid to be sterilized flows in an axial direction; an inflow port through which the fluid flows into the cylindrical body; an outflow port which is provided on an outer circumference of the cylindrical body and through which the fluid flows out; a first light source which is mounted on a substrate provided on an end section of the cylindrical body on a side opposite to the inflow port and which has a light emitting element emitting ultraviolet light toward the flow passage; and a first optical member which is composed of a cylindrical section and a leading end section, houses the first light source, and transmits, collects or scatters the ultraviolet light, wherein the cylindrical section is fitted to an inner wall of the cylindrical body, and a boundary section between the cylindrical section and the leading end section is disposed at a position coinciding with an end surface of the outflow port on a side close to the first light source or at a position where the boundary section protrudes beyond the end surface on the side close thereto.

In the fluid sterilization device in accordance with the present invention, ultraviolet light emitted from the first light source is transmitted, collected, or scattered at the first optical member housing the first light source. Then, sterilization progresses by the ultraviolet light irradiated to a fluid passing through the flow passage.

In the device, the cylindrical section of the first optical member is fitted to the inner wall of the cylindrical body such that there is almost no gap so as to prevent ultraviolet light irradiation efficiency from decreasing due to air bubbles generated inside because of the orientation of the device placement. Further, the boundary section between the cylindrical section and the leading end section of the first optical member is disposed at a position that coincides with the end surface of the outflow port on the side close to the first light source or a position where the boundary section protrudes beyond the aforementioned end surface thereby to guide a fluid hitting the leading end section toward the outflow port. This enables the device to improve the sterilization effect while suppressing the generation of air bubbles.

In the fluid sterilization device in accordance with the present invention, the cylindrical body and the cylindrical section of the first optical member preferably have a round cylindrical shape.

In the present invention, the cylindrical body of the fluid sterilization device and the cylindrical section of the first optical member are both formed into a round cylindrical shape. This enables the device to suppress the generation of air bubbles inside thereby to permit smooth flow of a fluid. Further, the inner diameter of the cylindrical body and the outer diameter of the cylindrical section are matched, thus making it possible to easily fit the cylindrical section to the cylindrical body without a gap.

Further, in the fluid sterilization device in accordance with the present invention, the leading end section of the first optical member preferably has a planar shape.

In the present invention, the leading end section of the first optical member has a planar shape. The ultraviolet light emitted from the first light source is transmitted through the leading end section and collected or scattered. This enables the device to evenly irradiate a fluid in the flow passage with the ultraviolet light.

Further, in the fluid sterilization device in accordance with the present invention, the boundary section is preferably chamfered.

In the case where the leading end section of the first optical member has a planar shape, the chamfered boundary section is disposed at a position that coincides with the end surface of the outflow port on the side close to the first light source or a position where the boundary section protrudes beyond the aforementioned end surface. This enables the device to guide a fluid toward the outflow port while suppressing the generation of air bubbles.

In the fluid sterilization device in accordance with the present invention, the leading end section of the first optical member preferably has a curved surface shape protruding toward the flow passage.

The leading end section of the first optical member may have a curved surface shape (round shape). In this case, the leading end section can collect the ultraviolet light emitted from the first light source to a specific portion of the flow passage. In other words, the device can obtain desired light distribution by changing the leading end section of the optical member according to a purpose.

Further, in the fluid sterilization device in accordance with the present invention, preferably, the cylindrical body has a notched section in the inner wall of an end section adjacent to a first light source, and the first optical member is fixed through a sealing member placed in the notched section.

The cylindrical body has the notched section in the inner wall of the end section, so that the device can provide the notched section with the sealing member. The device can fix the first optical member by the sealing member, and can also prevent a fluid from entering to a first light source side.

Further, in the fluid sterilization device in accordance with the present invention, preferably, the cylindrical body has a notched section at a position corresponding to the boundary section of the cylindrical body, and the first optical member is fixed through a sealing member placed in the notched section.

The cylindrical body in the present invention may have a notched section at a position corresponding to the boundary section of the first optical member. In this case also, the notched section can be provided with a sealing member, thus enabling the device to prevent the entry of a fluid even if there is a small gap between the cylindrical body and the first optical member (the cylindrical section).

In addition, the fluid sterilization device in accordance with the present invention preferably has, inside the first optical member, a reflector which is placed on the substrate in such a manner as to surround the first light source to reflect the ultraviolet light on an inner surface thereof so as to guide the ultraviolet light toward the flow passage.

With this arrangement, the ultraviolet light emitted from the first light source is reflected on the inner surface of the reflector. Consequently, the device can distribute the ultraviolet light to an arbitrary place and to intensively irradiate a part having a high flow velocity in a flow passage with the ultraviolet light. This enables the device to further improve the sterilization efficiency of a fluid.

Further, in the fluid sterilization device in accordance with the present invention, a plurality of outflow ports are preferably provided at positions that are symmetrical in a circumferential direction of the cylindrical body.

According to the present invention, a plurality of outflow ports are provided at positions that are symmetrical in the circumferential direction of the cylindrical body. This enables the device to discharge a sterilized fluid without stagnation in the vicinity of the outflow ports.

Further, in the fluid sterilization device in accordance with the present invention, the inflow port preferably has a truncated cone section, a diameter of which increases toward the cylindrical body, between the inflow port and the cylindrical body.

Providing the truncated cone section on the fluid inflow port side enables the device to allow a fluid to flow into the cylindrical body smoothly to some extent. In addition, the device can suppress the generation of air bubbles on the inflow port side.

Further, in the fluid sterilization device in accordance with the present invention, the inflow port is preferably provided on an outer circumference of the cylindrical body.

The inflow port of a fluid provided on the outer circumference of the cylindrical body makes it possible for the device to be used according to a purpose as a device having a so-called U-shaped flow passage.

Further, preferably, the fluid sterilization device in accordance with the present invention includes: a second light source which is mounted on a substrate provided on an end section of the cylindrical body on a side close to the inflow port and which has a light emitting element emitting ultraviolet light toward the flow passage; and a second optical member which is composed of a cylindrical section and a leading end section, houses the second light source, and transmits, collects or scatters the ultraviolet light, wherein the cylindrical section is fitted to an inner wall of the cylindrical body, and a boundary section between the cylindrical section and the leading end section is disposed at a position coinciding with an end surface of the inflow port on a side close to the second light source, or protrudes beyond the aforementioned end surface on the side close thereto.

In the device, when the inflow port is provided on the outer circumference of the cylindrical body, the second light source can be provided at the end section on the side close to the inflow port. Further, in the device, a fluid is irradiated by the ultraviolet light of the second light source from the inflow port side, and the fluid is irradiated by the ultraviolet light of the first light source from the outflow port side, thus improving sterilization efficiency.

In addition, the second optical member provided for the second light source is also placed such that the cylindrical section is fitted to the inner wall of the cylindrical body, and the boundary section is placed at a position that coincides with the end surface of the inflow port on the side close to the second light source or a position where the boundary section protrudes beyond the aforementioned end surface. This enables the device to suppress the generation of air bubbles on the inflow port side.

### Brief Description of Drawings

FIG. 1A is a side view of a fluid sterilization device according to an embodiment of the present invention;
FIG. 1B is a rear view of the fluid sterilization device according to the embodiment of the present invention;
FIG. 2 is a sectional view of the fluid sterilization device of FIG. 1B (a first embodiment) taken along II-II;
FIG. 3A is an enlarged view of a light source module unit of FIG. 2;
FIG. 3B is a diagram illustrating a modified form of a housing of FIG. 2;
FIG. 4 is a sectional view of a fluid sterilization device according to a second embodiment;
FIG. 5 is an enlarged view of a light source module unit of FIG. 4; and
FIG. 6 is a sectional view of a fluid sterilization device according to a third embodiment.

### Description of Embodiments

The following will describe embodiments of a fluid sterilization device in accordance with the present invention.

### [First Embodiment]

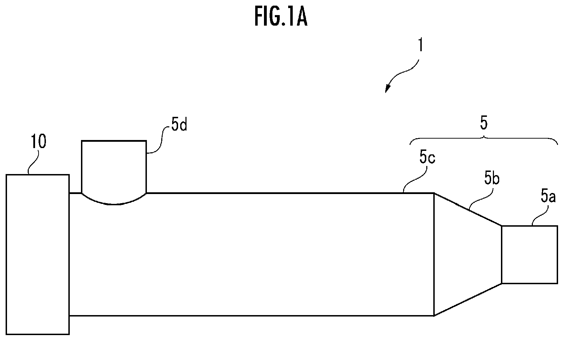

First, FIG. 1A presents a side view of a first embodiment of a fluid sterilization device in accordance with the present invention. A fluid sterilization device 1 is a device adapted to irradiate ultraviolet light to a fluid flowing through a flow passage thereby to sterilize the fluid. The fluid sterilization device 1 is used in a water purifier, a water heater, a water server, an industrial cooling water circulation device, and the like.

The fluid sterilization device 1 is provided with a housing 5 which has a flow passage and serves as a fluid sterilization unit, and a light source module unit 10, which includes an LED (Light Emitting Diode) as a light source.

The housing 5 has a straight pipe shape with a diameter of 31.8 mm (the inner diameter being 29.4 mm) and a flow passage (sterilization section) length of 100 mm, and is configured such that a fluid to be sterilized flows in the long axis direction of a cylindrical body 5c. Although the material of the cylindrical body 5c varies depending on the purpose of the fluid sterilization device 1, the material in this case is stainless steel. The fluid flows into the cylindrical body 5c through an inflow port 5a (the inner diameter being 12.7 mm) attached to one end of the cylindrical body 5c in the axial direction, and flows out through an outflow port 5d (the inner diameter being 12.7 mm) provided on the outer circumference of the cylindrical body 5c. The flow rate is 0.5 to 5 (L/min).

Further, the housing 5 has a truncated cone section 5b, the diameter of which increases toward the cylindrical body 5c, between the inflow port 5a and the cylindrical body 5c. A fluid flowing in through the inflow port 5a smoothly expands without rapidly expanding due to the presence of the truncated cone section 5b. Therefore, the fluid does not stagnate in a boundary region after the flow passage between the inflow port 5a and the cylindrical body 5c is expanded, thus making it possible to suppress the generation of air bubbles.

A rectifying plate (not illustrated) may be provided in the vicinity of the boundary section between the truncated cone section 5b and the cylindrical body 5c. In this case, a fluid that flows in through the inflow port 5a passes through the rectifying plate to reach the flow passage of the cylindrical body 5c. The rectifying plate is a plate made of metal or fluororesin and has a plurality of holes penetrating in the axial direction of the cylindrical body 5c. By passing through the rectifying plate, the fluid levels the flow velocity thereof when flowing into the flow passage of the cylindrical body 5c. Therefore, the ultraviolet light will be evenly irradiated to the fluid, thus improving the sterilization performance.

The housing 5 illustrated in FIG. 1A is called an L-shaped pipe because of the layout of the inflow port 5a and the outflow port 5d. The housing 5 may be formed in a so-called U-shaped pipe by providing the inflow port 5a on the outer circumference of the cylindrical body 5c. Further, a detachable inflow unit may be attached to an end section (on the side opposite to the light source module unit 10) of the cylindrical body 5c to form an L-shaped flow passage.

In the example of FIG. 1A, the quantity of the outflow port 5d is one, but a plurality of outflow ports may be provided on the outer circumference of the cylindrical body 5c. In this case, the outflow ports are preferably placed at positions symmetrical in the circumferential direction of the cylindrical body 5c. For example, if three outflow ports are provided, then the outflow ports may be placed at 120-degree intervals.

The light source module unit 10 is attached to the end section of the cylindrical body 5c on the opposite side to the inflow port 5a. As will be described in detail later, a light source, a substrate, a reflector, a quartz cap, and the like are housed inside the light source module unit 10.

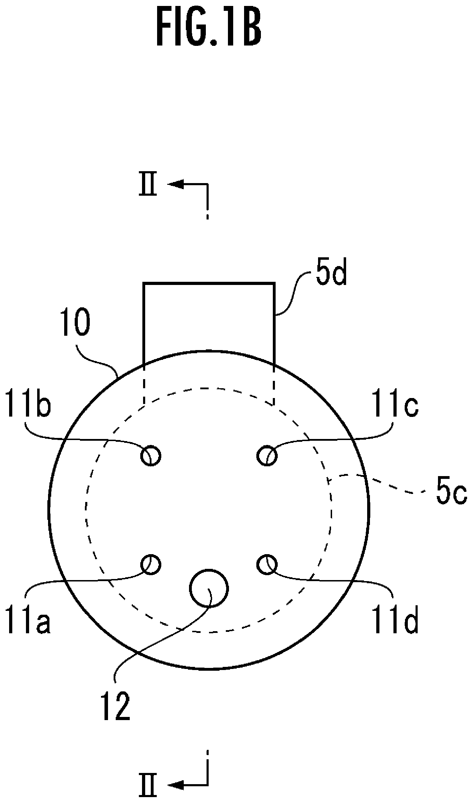

FIG. 1B is a view (a rear view) illustrating the fluid sterilization device 1 observed from the direction of the light source module unit 10. Here, the outflow port 5d of the cylindrical body 5c protrudes upward. If there are two outflow ports, then a second outflow port will protrude at a position symmetrical in the circumferential direction of the cylindrical body 5c, i.e., protrude downward.

Holes 11a to 11d of the light source module unit 10 (on the rear side) are holes for screws for attaching a light source substrate, which will be described later. Further, a hole 12 is a hole for a harness that connects the wiring of the substrate to an external power source. If a metal heat sink (not illustrated) is provided on the back surface side of the substrate (the side without the light emitting surface of the light source), the heat of the substrate can be dissipated.

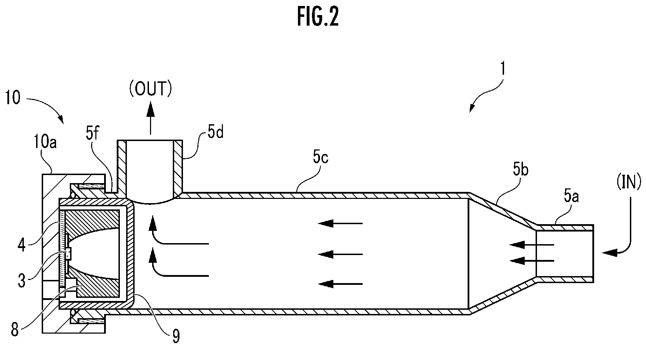

Next, FIG. 2 is a sectional view of the fluid sterilization device 1 of FIG. 1B taken along II-II.

A female screw is formed in a connection port 5f of the cylindrical body 5c that is adjacent to the light source module unit 10. Further, in the light source module unit 10, a male screw is formed on the inner wall of a frame body 10a. Thus, the light source module unit 10 can be screwed to the connection port 5f. The relationship between the male screw and the female screw may be reversed, i.e., the connection port 5f may have the male screw.

A fluid entering through the inflow port 5a and reaching the flow passage of the cylindrical body 5c travels toward the outflow port 5d, and flows outside the housing 5. At this time, the fluid is exposed in the flow passage to the ultraviolet light emitted from a light source 3 (corresponding to the "first light source" in the present invention) inside the light source module unit 10, and the fluid is thereby sterilized.

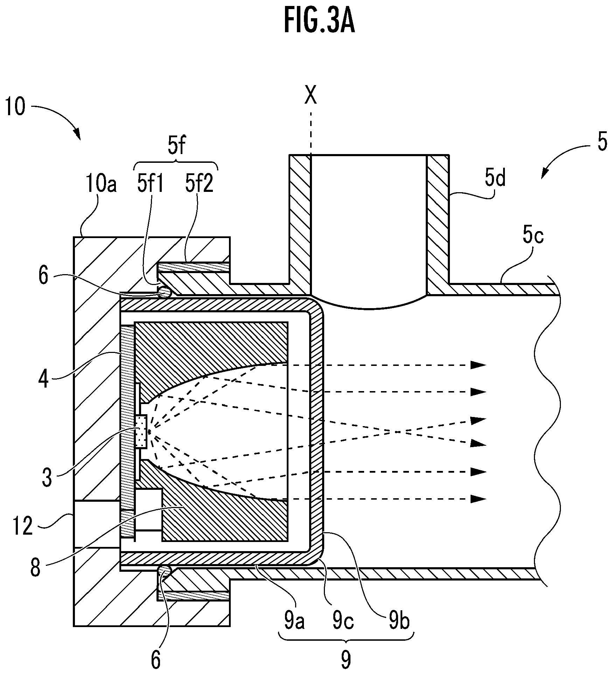

Next, FIG. 3A is an enlarged view of the light source module unit 10 of FIG. 2.

The ultraviolet light emitted from the light source 3 has a wavelength having a sterilization effect or a wavelength at which a chemical substance is decomposed. The wavelength is, for example, in the range of 240 to 280 nm. The light source 3 is an ultraviolet LED, and as illustrated in the drawing, one light source 3 is mounted on the front surface side of a substrate 4. A plurality of ultraviolet LEDs can also be lined up to form the light source.

The substrate 4 is desirably made of a metal such as copper or aluminum, which has excellent heat dissipation. Power is supplied to the light source 3 through the substrate 4. Further, the substrate 4 is in contact with the frame body 10a of the light source module unit 10 on the rear surface side (the side opposite to the light emitting surface of the light source 3) and is fixed with screws (not illustrated).

Further, a reflector 8 is placed on the front surface side of the substrate 4 in such a manner as to surround the light source 3. The reflector 8 is a spheroidal or paraboloidal reflecting mirror. The ultraviolet light emitted from the light source 3 is reflected on the inner surface of the reflector 8 and distributed to an arbitrary place, and travels toward the flow passage of the cylindrical body 5c. Consequently, the ultraviolet light is intensively irradiated to a portion of the flow passage where the flow velocity of the fluid is high, so that the sterilization efficiency of the fluid can be improved.

The ultraviolet light reflected by the reflector 8 passes through a quartz cap 9 (corresponding to the "first optical member" in the present invention) installed in such a manner as to cover the reflector 8. The quartz cap 9 is a member that is made of quartz glass, which has a refractive index larger than that of air, processed to a substantially uniform thickness, and composed of a cylindrical section 9a having a round cylindrical shape and a leading end section 9b having a non-round-cylindrical shape. The leading end section 9b has a planar shape or a curved surface shape connected or extended to the cylindrical section 9a. The boundary part between the cylindrical section 9a and the leading end section 9b is the boundary section 9c, which is chamfered.

The cylindrical body 5c may have a polygonal square cylindrical shape. However, as in the present embodiment, by forming both the cylindrical body 5c and the cylindrical section 9a into a round cylindrical shape, the generation of air bubbles inside can be suppressed so as to smooth the flow of a fluid. In addition, by matching the inner diameter of the cylindrical body 5c with the outer diameter of the cylindrical section 9a, the cylindrical section 9a can be easily fitted into the cylindrical body 5c with almost no gap. If both the cylindrical body 5c and the cylindrical section 9a are formed to have a rectangular cylindrical shape, then the ridgeline portions are desirably chamfered.

The cylindrical section 9a is fitted to the inner wall of the cylindrical body 5c with almost no gap (although there is a gap of approximately 0.7 mm). Further, the leading end section 9b, through which ultraviolet light passes, is formed into a planar shape with respect to an open end surface of the reflector 8. As illustrated, when ultraviolet light passes through the quartz cap 9 (the leading end section 9b), the ultraviolet light is distributed to an arbitrary place by refraction, and hits a portion in the flow passage where the flow velocity of the fluid is high. Consequently, the ultraviolet light is efficiently irradiated to the fluid in the flow passage.

When using the fluid sterilization device 1, the front surface side of the quartz cap 9 is filled with a fluid having a refractive index that is larger than that of air. As will be described in detail later, the leading end section 9b of the quartz cap 9 may have a convex shape or any other shape with respect to the open end surface of the reflector 8. The reflector 8 is not an essential component of the present invention, and the light distribution of ultraviolet light may be controlled by the shape of the leading end section 9b.

Further, the quartz cap 9 is disposed such that the boundary section 9c is positioned so as to protrude beyond an end surface (inner wall) X of the outflow port 5d on the side close to the light source module unit 10. Consequently, the fluid that circulates through the cylindrical body 5c and hits the leading end section 9b of the quartz cap 9 is guided toward the outflow port 5d while hardly flowing into the gap between the cylindrical body 5c and the quartz cap 9. The quartz cap 9 may be placed at a position where the boundary section 9c coincides with an end surface X.

When the fluid sterilization device 1 is placed vertically (the light source module unit 10 being on the upper side), there is a concern that air bubbles are generated in the gap and the fluid that has reached the vicinity of the outflow port 5d is not sufficiently irradiated with ultraviolet light. However, the quartz cap 9 placed as described above enables the fluid sterilization device 1 to suppress the generation of air bubbles in the vicinity of the outflow port 5d, thus improving the sterilization efficiency of the fluid. In order to further reduce the gap, the cylindrical section 9a of the quartz cap 9 may be covered with a band-shaped silicone sheet.

Further, as illustrated, a triangular groove 5f1 is formed in the inner wall of the end section of the connection port 5f. The presence of the triangular groove 5f1 creates a space between the cylindrical section 9a of the quartz cap 9 and the connection port 5f, so that an O-ring 6 (corresponding to the "sealing member" in the present invention) is placed. Consequently, the sealing function of the O-ring 6 can prevent a fluid from entering the light source module unit 10. A connection section 5f2 in the drawing is a section where a female screw of the connection port 5f is formed.

The O-ring 6 is formed of a fluorine-based material, and may deteriorate when exposed to ultraviolet light. However, this position is outside the quartz cap 9 and also outside the reflector 8. The O-ring 6 is hardly irradiated with ultraviolet light, so that deterioration of the O-ring 6 can be prevented.

Thus, the light source module unit 10 is smaller than a conventional mechanism that adjusts light distribution by stacking lenses. Consequently, the fluid sterilization device 1 can make the entire device smaller. In addition, the quartz cap 9 is fitted like a cap around the outer circumference of the reflector 8, thus providing the advantage of being easy to replace. The light source module unit 10 may have a plurality of light sources and reflectors on a single substrate, which is covered by a single quartz cap.

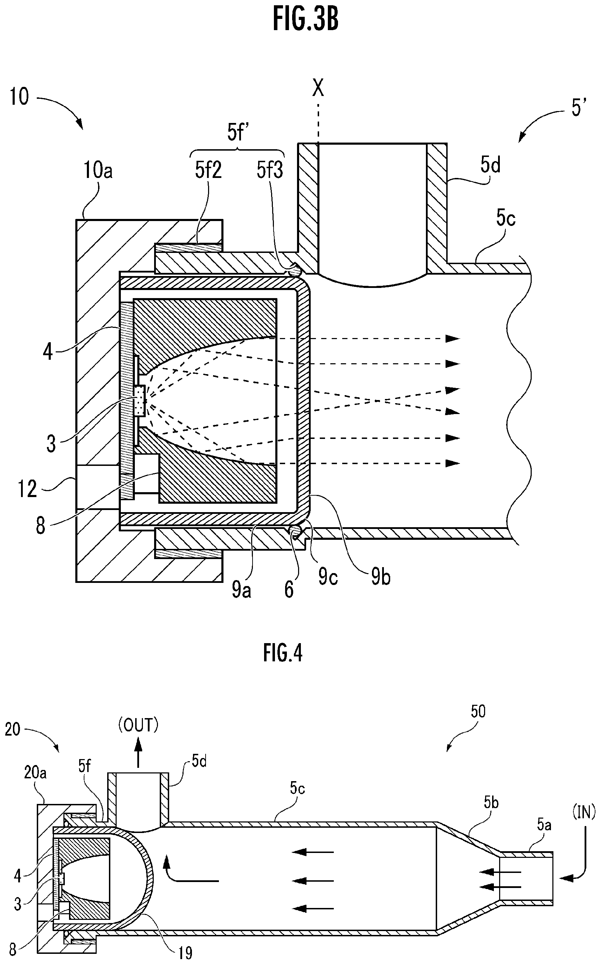

Referring now to FIG. 3B, a modified form (housing 5') of the aforementioned housing 5 will be described.

The housing 5 illustrated in FIG. 3A is sealed by the O-ring 6 of the triangular groove 5f1. However, a slight gap was formed between the cylindrical section 9a of the quartz cap 9 and the connection port 5f, thus allowing a fluid to enter. In this respect, in the housing 5' (a connection port 5f) of the modified form, a triangular groove 5f3 is provided at a position corresponding to the boundary section 9c of the quartz cap 9 to prevent the fluid from entering the gap.

As illustrated, the triangular groove 5f3 of the connection port 5f' is provided in the vicinity of the end surface X of the cylindrical body 5c on the side close to the light source module unit 10. Further, the O-ring 6 is disposed at the space resulting from the triangular groove 5f3. In this case also, the entry of the fluid into the light source module unit 10 can be prevented by the sealing function of the O-ring 6. Obviously, the quartz cap 9 is preferably placed such that the boundary section 9c is positioned so as to protrude beyond the end surface X of the outflow port 5d.

### [Second Embodiment]

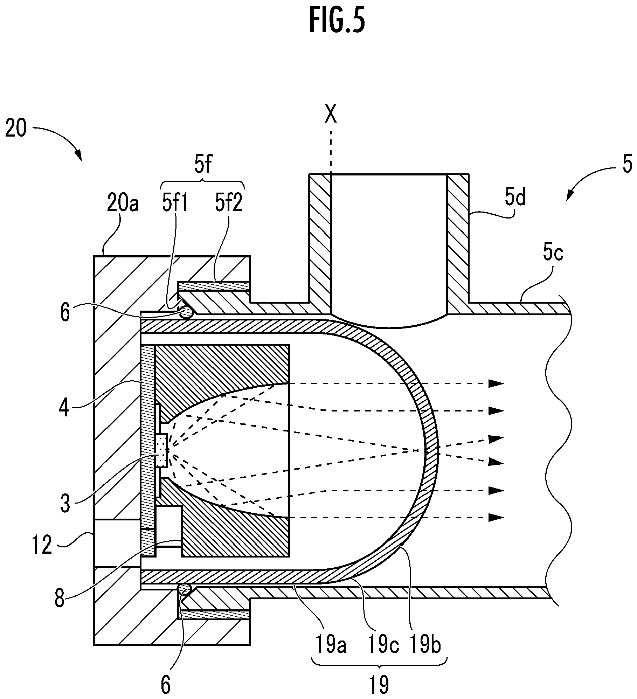

Referring now to FIG. 4 and FIG. 5, a second embodiment of the fluid sterilization device in accordance with the present invention will be described.

FIG. 4 is a sectional view of a fluid sterilization device 50 of the second embodiment. The side view and the rear view of the fluid sterilization device 50 are the same as FIG. 1A and FIG. 1B, and are therefore omitted. In the following description, the like components as those of the first embodiment will be assigned the like reference numerals and the descriptions thereof will be omitted.

As illustrated, a female screw is formed in a connection port 5f of the end section of a cylindrical body 5c on the side adjacent to a light source module unit 20. In the light source module unit 20, a male screw is formed on the inner wall of a frame body 20a, so that the light source module unit 20 can be screwed to the connection port 5f.

A fluid entering through an inflow port 5a and reaching the flow passage of the cylindrical body 5c travels toward an outflow port 5d, and flows outside a housing 5. At this time, the fluid is exposed in the flow passage to the ultraviolet light emitted from a light source 3 inside the light source module unit 20, and the fluid is thereby sterilized. The leading end section of a quartz cap 19 of the light source module unit 20 has a curved surface shape (round shape) protruding toward the flow passage.

Next, FIG. 5 is an enlarged view of the light source module unit 20 of FIG. 4.

The light source module unit 20 includes a light source 3, a substrate 4, a reflector 8, and a quartz cap 19 inside a frame body 20a. The placement of the members constituting the light source module unit 20 is the same as that in the light source module unit 10 (refer to FIG. 2B) described above.

The ultraviolet light emitted from the light source 3 and reflected on the inner surface of the reflector 8 passes through the quartz cap 19 attached in such a manner as to cover the reflector 8. The quartz cap 19 is also quartz glass having a substantially uniform thickness. The ultraviolet light is refracted when passing through the quartz cap 19, and the ultraviolet light is distributed to an arbitrary place in the same manner as with the quartz cap 9 (having the planar shape) described above. Therefore, the quartz cap 19 is best suited when a fluid in a certain region is to be intensively irradiated with ultraviolet light.

Here, too, a cylindrical section 19a having a round cylindrical shape is fitted to the inner wall of the cylindrical body 5c with almost no gap. Further, an O-ring 6 is disposed between the quartz cap 19 and the connection port 5f (the triangular groove 5f1), so that it is possible to prevent a fluid from entering the light source module unit 20.

In addition, the quartz cap 19 is placed such that a boundary section 19c is positioned so as to protrude beyond an end surface (the inner wall) X of the outflow port 5d on the side close to the light source module unit 20. Consequently, a fluid that circulates through the cylindrical body 5c and hits the leading end section 19b of the quartz cap 19 is guided toward the outflow port 5d while hardly flowing into the gap between the cylindrical body 5c and the quartz cap 19.

The quartz cap 19 may be placed at a position where the boundary section 19c coincides with the end surface X. The quartz cap 19 placed at this position enables the device to suppress the generation of air bubbles in the vicinity of the outflow port 5d, thus improving the sterilization efficiency of the fluid.

There are various other possible shapes for the quartz cap, each of which can be used according to the purpose of the device. For example, the leading end section through which ultraviolet light passes may be formed to be convex on both sides, or the leading end section may be flat on one side (on the flow passage side) and concave or convex on the other side (on the light source 3 side). Further, these can be used for different light distribution control. In the case of the quartz cap 19, a space is created between the quartz cap 19 and the reflector 8 (the inner surface), so that a concave lens or a convex lens may be disposed in the space to control the light distribution.

### [Third Embodiment]

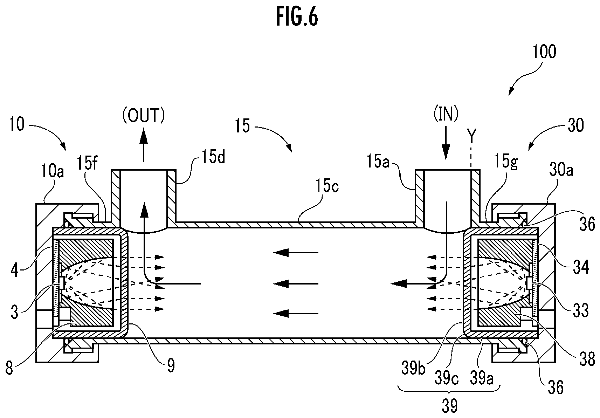

Lastly, with reference to FIG. 6, a third embodiment of the fluid sterilization device in accordance with the present invention will be described.

FIG. 6 is a sectional view of a fluid sterilization device 100 of the third embodiment. The fluid sterilization device 100 is provided with a housing 15 which has a flow passage and serves as a fluid sterilizing unit, and a light source module unit 10 and a light source module unit 30 that include LEDs as light sources.

The housing 15 is a so-called U-shaped pipe having an inflow port 15a and an outflow port 15d installed on the outer circumference of a cylindrical body 15c. A fluid flows into the cylindrical body 15c through the inflow port 15a (the inner diameter being 12.7 mm) and flows out through the outflow port 15d (the inner diameter being 12.7mm). The flow rate is 0.5 to 5 (L/min).

A light source module unit 10, which is the same as that in the first embodiment, is attached to a connection port 15f, which is one end section (on the left side in the drawing) of the cylindrical body 15c. Further, the light source module unit 30 is attached to a connection port 15g, which is the other end section (on the right side in the drawing) of the cylindrical body 15c.

A light source 33, a substrate 34, a reflector 38, and a quartz cap 39 are housed inside the light source module unit 30. A fluid is irradiated with ultraviolet light by the light source 33 (corresponding to the "second light source" in the present invention) of the light source module unit 30 immediately after the fluid flows into the housing 15 through the inflow port 15a.

The ultraviolet light emitted from the light source 33 is reflected on the inner surface of the reflector 38, becomes collimated light, and travels toward the flow passage of the cylindrical body 15c. Consequently, a fluid in the flow passage is uniformly irradiated with the ultraviolet light, so that the sterilization efficiency of the fluid can be improved. Further, the ultraviolet light reflected by the reflector 38 passes through the cap-shaped quartz cap 39 (corresponding to the "second optical member" in the present invention) attached in such a manner as to cover the reflector 38.

As with the quartz cap 9 in the first embodiment, the quartz cap 39 is a member that is made of quartz glass, which has a refractive index larger than that of air, processed to a substantially uniform thickness. The quartz cap 39 is composed of a cylindrical section 39a having a round cylindrical shape and a leading end section 39b having a non-round-cylindrical shape, and the boundary part between the cylindrical section 39a and the leading end section 39b is a boundary section 39c, which is chamfered.

The cylindrical section 39a is fitted to the inner wall of the cylindrical body 15c with almost no gap. Further, the leading end section 39b, through which ultraviolet light passes, is formed into a planar shape with respect to an open end surface of the reflector 38. When ultraviolet light passes through the quartz cap 39 (the leading end section 39b), the ultraviolet light is distributed to an arbitrary place by refraction, and hits a portion in the flow passage where the velocity of the fluid is high. Consequently, the ultraviolet light is efficiently irradiated to the fluid in the flow passage.

Further, the quartz cap 39 is disposed such that the boundary section 39c is positioned so as to protrude beyond an end surface (inner wall) Y of the inflow port 15a on the side close to the light source module unit 30. Consequently, the fluid that flows in through the inflow port 15a is guided toward the cylindrical body 15c while hardly flowing into the gap between the cylindrical body 15c (the connection port 15g) and the quartz cap 39. This enables the fluid sterilization device 100 to suppress the generation of air bubbles in the vicinity of the inflow port 15a, thus improving the sterilization efficiency of the fluid.

In addition, a space (a triangular groove) is provided between the quartz cap 39 and the connection port 15g of the cylindrical body 15c, and an O-ring 6 is disposed therein (refer to FIG. 3A). Consequently, the sealing function of the O-ring 6 can prevent a fluid from entering into the light source module unit 30.

Further, in the fluid sterilization device 100, a fluid that has passed through the cylindrical body 15c is irradiated with ultraviolet light by the light source 3 of the light source module unit 10 in the vicinity of the outflow port 15d. Thus, the sterilization efficiency can be further improved.

The light source module units 10 and 30 may adopt the aforementioned light source module unit 20 or the like according to a purpose. Further, the light source module unit 10 and the light source module unit 30 may adopt light source module units that are different from each other.

The embodiments described above are only examples, and can be changed when necessary to suit each application. The flow rate differs depending on each application, so that the size and the shape of the cylindrical body of the fluid sterilization device can be changed. In particular, the light source module unit in the present invention is not limited to a running water reactor adapted to be attached to a part of a water passage.

For example, by attaching the light source module unit to a server or a water storage tank having a dedicated connection port, ultraviolet light can be irradiated to a fluid in a container thereby to sterilize the fluid. The ultraviolet light may be irradiated to the fluid from the side surface of the container or irradiated from the top surface of the container. In addition to the above, the fluid sterilization device can also be used for surface sterilization, and for sterilization as a measure against water stains in bathrooms and mold.

The cross-sectional shape of the cylindrical body is not limited to a circular or oval shape, but may also be a polygonal shape. Depending on the cross-sectional area of the housing, three or more light source module units, for example, may be attached to the end sections of the housing. The number of light sources in a single light source module unit can also be changed when necessary, and the light sources can be arranged in a matrix pattern or the like, depending on the cross-sectional shape of the housing.

In a form in which the light source is disposed on one side of the flow passage, as in the fluid sterilization device 1, the direction in which a fluid flows is generally opposite to the irradiation direction of ultraviolet light, however, the direction may match the irradiation direction. The numbers and directions of the inflow ports and the outflow ports, the number of ultraviolet LEDs, and the like can be changed when necessary.

If the inner wall of the cylindrical body of the fluid sterilization device is made of polyvinyl chloride, the inner wall may be coated with an ultraviolet light reflecting material or an ultraviolet light absorbing material to prevent deterioration of the polyvinyl chloride caused by ultraviolet light. As the ultraviolet light reflecting material, a fluororesin such as PTFE, aluminum, or the like can be used. Further, as the ultraviolet light absorbing material, stainless steel or the like can be used.

1, 50, 100...fluid sterilization device; 3, 33...light source; 4, 34...substrate; 5, 5', 15...housing; 5a, 15a...inflow port; 5b...truncated cone section; 5c, 15c...cylindrical body; 5d, 15d...outflow port; 5f, 5f, 15f, 15g...connection port; 6, 36...0-ring; 8, 38...reflector; 9, 19, 39...quartz cap; 9a, 19a, 39a...cylindrical section; 9b, 19b, 39b...leading end section; 9c, 19c, 39c...boundary section; 10, 20, 30...light source module unit; 10a, 20a...frame body; 11a to 11d...hole (for screw); and 12...hole (for harness).

## Claims

1. A fluid sterilization device comprising:
a cylindrical body having a flow passage through which a fluid to be sterilized flows in an axial direction;
an inflow port through which the fluid flows into the cylindrical body;
an outflow port which is provided on an outer circumference of the cylindrical body and through which the fluid flows out;
a first light source which is mounted on a substrate provided on an end section of the cylindrical body on a side opposite to the inflow port and which has a light emitting element emitting ultraviolet light toward the flow passage; and
a first optical member which is composed of a cylindrical section and a leading end section, houses the first light source, and transmits, collects or scatters the ultraviolet light,
wherein the cylindrical section is fitted to an inner wall of the cylindrical body, and a boundary section between the cylindrical section and the leading end section is disposed at a position coinciding with an end surface of the outflow port on a side close to the first light source or at a position where the boundary section protrudes beyond the end surface on the side close thereto.

2. The fluid sterilization device according to claim 1, wherein the cylindrical body and the cylindrical section of the first optical member have a round cylindrical shape.

3. The fluid sterilization device according to claim 1 or 2, wherein the leading end section of the first optical member has a planar shape.

4. The fluid sterilization device according to claim 3, wherein the boundary section is chamfered.

5. The fluid sterilization device according to claim 1 or 2, wherein the leading end section of the first optical member has a curved surface shape protruding toward the flow passage.

6. The fluid sterilization device according to any one of claims 1 to 5,
wherein the cylindrical body has a notched section in an inner wall of an end section on the first light source side, and
the first optical member is fixed through a sealing member placed in the notched section.

7. The fluid sterilization device according to any one of claims 1 to 5,
wherein the cylindrical body has a notched section at a position corresponding to the boundary section of the cylindrical body, and
the first optical member is fixed through a sealing member placed in the notched section.

8. The fluid sterilization device according to any one of claims 1 to 7, having, inside the first optical member, a reflector which is placed on the substrate in such a manner as to surround the first light source to reflect the ultraviolet light on an inner surface thereof so as to guide the ultraviolet light toward the flow passage.

9. The fluid sterilization device according to any one of claims 1 to 8, wherein a plurality of the outflow ports are provided at positions that are symmetrical in a circumferential direction of the cylindrical body.

10. The fluid sterilization device according to any one of claims 1 to 9, wherein the inflow port has a truncated cone section, a diameter of which increases toward the cylindrical body, between the inflow port and the cylindrical body.

11. The fluid sterilization device according to any one of claims 1 to 9, wherein the inflow port is provided on an outer circumference of the cylindrical body.

12. The fluid sterilization device according to claim 11, including:
a second light source which is mounted on a substrate provided on an end section of the cylindrical body on a side close to the inflow port and which has a light emitting element emitting ultraviolet light toward the flow passage; and
a second optical member which is composed of a cylindrical section and a leading end section, houses the second light source, and transmits, collects, or scatters the ultraviolet light,
wherein the cylindrical section is fitted to an inner wall of the cylindrical body, and a boundary section between the cylindrical section and the leading end section is disposed at a position coinciding with an end surface of the inflow port on a side close to the second light source, or protrudes beyond the end surface on the side close thereto.
